# EUROPEAN PATENT SPECIFICATION

(11) **EP 3 422 950 B1**
(45) Date of publication and mention of the grant of the patent: **08.04.2020**
(21) Application number: 17704784.2
(22) Date of filing: 16.02.2017
(51) Int. Cl.: A61B 6/04, A61B 6/00, A61B 6/02, A61B 6/03

(54) **HYBRID X-RAY AND GAMMA IMAGING SYSTEM**
HYBRIDES RÖNTGEN- UND GAMMA-ABBILDUNGSSYSTEM
SYSTÈME D'IMAGERIE HYBRIDE À RAYONS X ET GAMMA

(30) Priority: 03.03.2016 EP 16158500
(43) Date of publication of application: 09.01.2019
(73) Proprietor: Koninklijke Philips N.V., 5656 AG Eindhoven (NL)
(72) Inventor: WIECZOREK, Herfried Karl, 5656 AE Eindhoven (NL); GOEDICKE, Andreas, 5656 AE Eindhoven (NL); STEGEHUIS, Herman, 5656 AE Eindhoven (NL); JACOBS, Johannes Wilhelmus Maria, 5656 AE Eindhoven (NL)
(74) Representative: Schaapman, Maaike Ruth
(86) International application number: PCT/EP2017/053456
(87) International publication number: WO 2017/148706

(56) References cited:
- DE-A1-102011 083 853
- JP-A- 2007 202 976
- US-A1- 2010 290 584

## Description

### FIELD OF THE INVENTION

The invention relates to the detection of x-ray and gamma quanta. It finds application in the medical field, more particularly in the fields of medical imaging and medical interventions, and may be used for example to provide an x-ray image and a corresponding nuclear image of a region of interest.

### BACKGROUND OF THE INVENTION

In various medical imaging procedures it is beneficial to provide both an x-ray image and a nuclear image of a region of interest. The x-ray image typically provides structural information indicative of the anatomy of the region of interest. The nuclear image, defined herein to mean an image indicative of radiotracer distribution in an object, is generated based on detected gamma quanta. The nuclear image may for example be a gamma scintigraphy or a SPECT image and typically provides functional, or physiological information relating to the region of interest. Together the two different image types can be used to improve the identification of an underlying pathology during a medical investigation.

Various medical procedures also benefit from a combination of x-ray and nuclear imaging. Selective internal radiation therapy, or SIRT, is one such medical procedure in which radiation is used to treat cancer. SIRT is often used for non-resectable cancers, i.e. cancers that cannot be treated surgically, and involves injecting microspheres of radioactive material into the arteries that supply the tumor. Liver tumors or metastases are often treated in this way. However, in delivering such therapy, a number of workflow steps are required in order to prevent potential side effects. These steps may include the closure of atypical lung and gastrointestinal shunts before injection of Yttrium-90 -containing microspheres. This prevents radiation ulcers which might otherwise be triggered by extra-hepatic localization of administered micro-spheres. For this purpose, catheter-based vessel coiling is performed under x-ray guidance during a minimally-invasive procedure. Afterwards, the remaining shunt level towards lungs and gastrointestinal area may be controlled by injection of Technetium ^{99m}Tc albumin aggregated, i.e. Tc-labeled MAA, into both main liver arteries followed by planar gamma imaging. During this procedure the patient is typically repeatedly transported between a cath lab and SPECT imaging room.

A need therefore exists for imaging systems that are capable of providing both a nuclear image and an x-ray image.

Document US2009/0016488A1 describes a medical diagnostic system having two c-arms which are adjustable with the aid of two drive means and serve as retaining devices for one medical measuring system in each case. The first measuring system is an x-ray measuring system comprising an x-ray emitter and an x-ray detector and has a high spatial resolution. The second measuring system is a nuclear medicine measuring system for visualizing tissue functions. Medical diagnoses and interventions are possible based on image information generated by both measuring systems.

A document DE 10 2011 083 853 A1 discloses an imaging arrangement that includes a C-arm for detecting anatomical image data, and a SPECT-measuring probe for detecting a SPECT signal emitted by an injected and metabolized SPECT tracer, to detect functional image data. A tracking system is provided for position determination and for correlating a position of the measuring probe and position of the C-arm. A calculation unit is provided for calculating and representing a fused image data set in which the functional data is superimposed to the anatomical data. The measuring probe is designed as a gamma-camera.

However, in the field of medical imaging, and in the field of medical procedures, there remains a need for improved imaging systems that are capable of providing both a nuclear image and an x-ray image.

### SUMMARY OF THE INVENTION

It is an object of the invention to provide a system for providing both a nuclear image and an x-ray image of a region of interest. Thereto a medical imaging arrangement is provided.

The medical imaging arrangement includes an x-ray source, an x-ray detector, an x-ray c-arm, and a gamma camera. The x-ray source is attached to a first portion of the x-ray c-arm and the x-ray detector is attached to a second portion of the x-ray c-arm. The x-ray source and the x-ray detector are so-positioned in order to measure x-ray transmission along a path between the x-ray source and the x-ray detector. Moreover, the gamma camera is movable along a trajectory that intersects the path between the x-ray source and the x-ray detector. Advantageously, because the gamma camera can be moved along a trajectory that intersects the path between the x-ray source and the x-ray detector, the images generated by the x-ray source and detector and the gamma camera are in close correspondence.

According to one aspect the imaging arrangement includes a patient support pallet. The patient support pallet is translatable along a longitudinal axis that passes lengthwise through the patient support pallet. Moreover the path between the x-ray source and the x-ray detector is arranged transversely with respect to the longitudinal axis. This arrangement provides improved imaging access around an object supported by the patient support pallet.

According to another aspect, in the imaging arrangement the trajectory intersects the path between the x-ray source and the x-ray detector at a point that is closer to the x-ray source than to the x-ray detector. Advantageously, in this positon the x-ray source-detector arrangement has a narrow field of view and thus sequential x-ray - nuclear imaging can be achieved with minimal movement of the gamma detector. Also, simultaneous x-ray - nuclear imaging can be achieved with a minimally-offset gamma detector.

Other aspects of the invention are described by the dependent claims.

### BRIEF DESCRIPTION OF THE FIGURES

Fig. 1 illustrates a first embodiment of a medical imaging arrangement that includes an x-ray source 111, an x-ray detector 112, an x-ray c-arm 113, and a gamma camera 114.
Fig. 2 illustrates a second embodiment of a medical imaging arrangement that includes an x-ray source 211, an x-ray detector 212, and an x-ray c-arm 213.
Fig. 3 illustrates a third embodiment of a medical imaging arrangement that includes an x-ray source 311, an x-ray detector 312, an x-ray c-arm 313, a gamma camera 314 and a patient support pallet 317.
Fig. 4 illustrates a fourth embodiment of a medical imaging arrangement that includes patient support pallet 417 that is translatable along a longitudinal axis 418, and in which gamma camera 414 is movable along trajectory 416 that is comprised within a plane that transversely intersects longitudinal axis 418.

### DETAILED DESCRIPTION OF THE INVENTION

As described above, the present invention provides a system for providing both a nuclear image and an x-ray image of a region of interest. Thereto a medical imaging arrangement is provided.

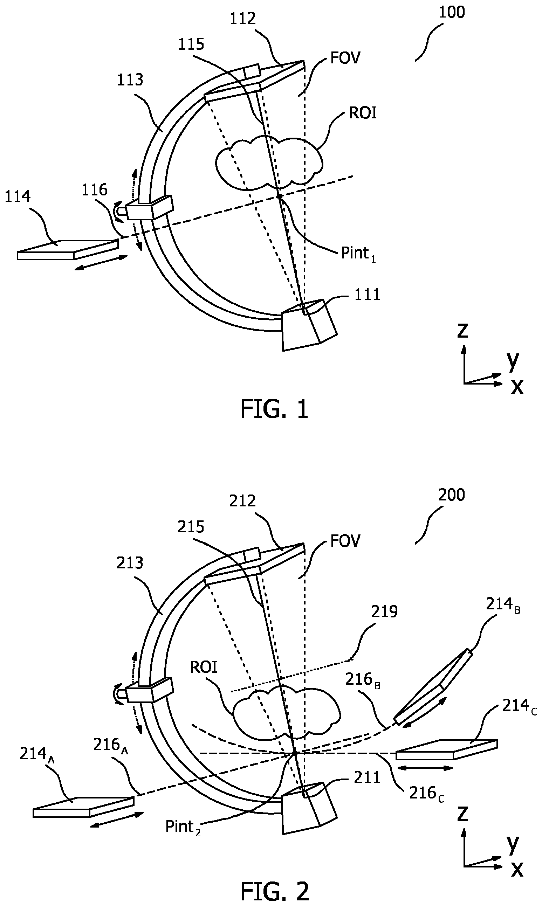

Fig. 1 illustrates a first embodiment of a medical imaging arrangement that includes an x-ray source 111, an x-ray detector 112, an x-ray c-arm 113, and a gamma camera 114. As illustrated in Fig. 1, x-ray source 111 is attached to a first portion of x-ray c-arm 113 and x-ray detector 112 is attached to a second portion of x-ray c-arm 113. The x-ray source and the x-ray detector are so-positioned in order to measure x-ray transmission along path 115 between the x-ray source and the x-ray detector. The field of view FOV of the x-ray source - x-ray detector arrangement in Fig. 1 is illustrated by the short dashed lines that comprise path 115. Moreover, gamma camera 114 is movable along trajectory 116 as indicated. Trajectory 116 may for example be defined by rails, or by an extendable member, or by grooves in a surface or by another support structure not shown in Fig. 1. Trajectory 116 intersects path 115 between x-ray source 111 and x-ray detector 112 at point Pinti.

Advantageously, because gamma camera 114 can be moved along a trajectory that intersects path 115 between x-ray source 111 and x-ray detector 112, gamma camera 114 can be used to generate a nuclear image that corresponds to the same region of interest ROI as that which is imaged by x-ray source 111 and x-ray detector 112. Because the trajectory and the path intersect one another, close correspondence between the nuclear image and the x-ray image can be achieved. Moreover, when the gamma camera has its own support structure, independent movement between the gamma camera and the x-ray c-arm can be achieved, thereby providing increased positioning flexibility.

Preferably, x-ray detector 112 in Fig. 1 is a flat panel x-ray detector array that includes an array of x-ray scintillator elements for generating x-ray scintillation light and a corresponding array of photodetectors arranged to capture the x-ray scintillation light. X-ray detector 112 may alternatively include a curved panel or a plurality of flat panel segments arranged around an arc to provide a curved geometry. X-ray source 111 can be a standard x-ray source, although it is also contemplated to use a dual energy source in this position. X-ray c-arm 113 is an otherwise standard x-ray c-arm and may be mounted in a fixed position or optionally arranged for movement as illustrated by the arrows near its support.

In addition to intersection point Pint₁, trajectory 116 in Fig. 1 may also include an offset position along the trajectory in which the gamma camera is not intercepted by path 115 between the x-ray source and detector. This may for example be to one end of trajectory 116 at which position the gamma camera is beyond the field of view FOV of the x-ray source 111 - x-ray detector 112 arrangement. The ability to move gamma camera 114 along trajectory 116 between point of intersection Pinti, and the offset position provides two possible nuclear imaging positions in which differing views and thus degrees of correspondence between the x-ray image and the nuclear image can be achieved. Clearly, additional nuclear imaging positions along trajectory 116 may also be provided. Moreover, the gamma camera may be positioned in the offset position where it is beyond the field of view FOV of the x-ray source and detector during x-ray imaging in order to prevent the gamma camera from obscuring or being affected by x-ray radiation emitted by x-ray source 111.

In-use, the arrangement of Fig. 1 may be used in a number of different modes. In one exemplary mode, x-ray source 111 - x-ray detector 112 arrangement may be used to generate single-shot or a continuous stream of x-ray images of region of interest ROI in order to perform a medical procedure. One such medical procedure is the catheter-based vessel coiling procedure outlined above. Having performed the medical procedure under x-ray guidance, gamma camera 114 may be moved along trajectory 116 to point Pinti where trajectory 116 intersects path 115. In this position gamma camera 114 can be used to generate a nuclear image of the ROI that closely corresponds to the x-ray image. Moreover, because point Pinti is close to region of interest ROI, a high number of gamma counts from the ROI will be detected by gamma camera 114, in short resulting in a higher resolution nuclear image. Thus, in this exemplary mode the x-ray and nuclear images may be generated sequentially. In another exemplary mode x-ray and nuclear images may be generated simultaneously with gamma camera 114 in the above-described offset position. In this mode the x-ray and gamma camera views of the ROI are clearly slightly different. Advantageously, in this simultaneous imaging configuration, no further adjustments of the imaging system positions are needed after an initial set-up phase.

Other gamma camera trajectories to that illustrated in Fig. 1 may also be used. Fig. 2 illustrates a second embodiment of a medical imaging arrangement that includes an x-ray source 211, an x-ray detector 212, and an x-ray c-arm 213. Moreover, multiple optional gamma camera trajectories 216_{A, B, C} for each of corresponding gamma cameras 214_{A, B,} c are also shown in Fig. 2. For example, trajectories 216_{A} and 216c are aligned with arbitrary axes y and x respectively, whereas trajectory 216_{B} is an arc that lies in the z-x plane. In a preferred configuration a single gamma camera exemplified by one of gamma camera 214_{A, B,} c is used, and this is movable along at least one of trajectories 216_{A, B, C}. Multiple gamma cameras may also be used in a similar manner. X-ray source 211 and corresponding x-ray detector 212 in Fig. 2 are so-positioned in order to measure x-ray transmission along path 215 between x-ray source 211 and x-ray detector 212. Moreover, each exemplary gamma camera 214_{A, B, C} is movable along its corresponding trajectory 116_{A, B, C} as indicated. Arc-shaped trajectory 216_{B} may for example be defined by rails, or by an extendable beam, or by grooves in a surface or by a c-arm not shown in in Fig. 1. Each of trajectories 216_{A, B, C} intersect path 215 between x-ray source 211 and x-ray detector 212 at point Pint₂. Optionally intersection point Pint₂ is closer to x-ray source 211 than to x-ray detector 212. Advantageously, in this positon the x-ray source - x-ray detector arrangement has a narrow field of view and thus sequential x-ray - nuclear imaging can be achieved with minimal movement of the gamma detector. Also, simultaneous x-ray - nuclear imaging can be achieved with a minimally-offset gamma detector.

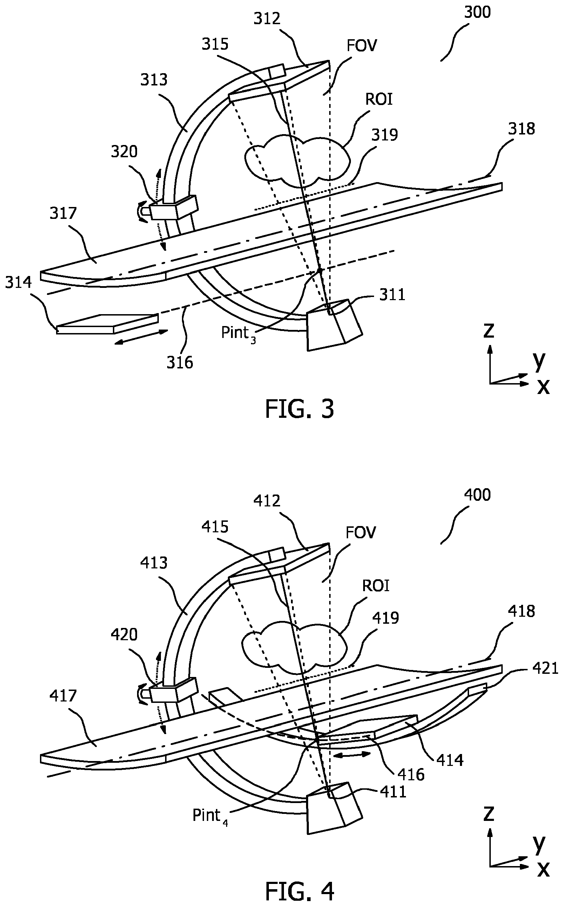

Fig. 3 illustrates a third embodiment of a medical imaging arrangement that includes an x-ray source 311, an x-ray detector 312, an x-ray c-arm 313, a gamma camera 314 and a patient support pallet 317. The x-ray source 311 and the x-ray detector 312 are positioned on x-ray c-arm 313 in order to measure x-ray transmission along path 315. Moreover, gamma camera 314 is movable along trajectory 316. Trajectory 316 intersects path 315 between x-ray source 311 and x-ray detector 312 at point Pint₃. Moreover patient support pallet 317 has a longitudinal axis 318 that passes lengthwise through the patient support pallet, and the patient support pallet 317 is translatable along longitudinal axis 318. Furthermore, path 315 between x-ray source 311 and x-ray detector 312 is arranged transversely with respect to longitudinal axis 318. Because the patient support pallet 317 is translatable along longitudinal axis 318, improved imaging access around an object supported by the patient support pallet can be achieved.

Preferably, trajectory 316 and longitudinal axis 318 in Fig. 3 are mutually parallel. This allows gamma camera 314 to be moved in harmony with the positon of an object supported by patient support pallet 317, thereby offering continuous nuclear imaging of the object during movement of the patient support pallet, for example during live x-ray imaging. In one configuration gamma camera 314 may be attached-to, or supported by patient support pallet 317, optionally via a gamma camera support structure. This allows the same portion of an object supported by patient support pallet 317 to be imaged by gamma camera 314 during translation of patient support pallet 317 along its longitudinal axis. This in turn improves image quality because a gamma image of the object can be continuously, i.e. even during translation of the object during x-ray imaging. Moreover, such a supporting arrangement ensures close correspondence between patient motion and motion of the gamma camera, thereby improving image quality. In another configuration gamma camera 314 is translatable with respect to patient support pallet 317. This permits an operator to either image a different portion of an object supported by patient support pallet 317, or to move gamma detector 314 to an offset position during x-ray imaging in order to prevent the gamma camera from obscuring or being affected by x-ray radiation as described above.

Fig. 4 illustrates a fourth embodiment of a medical imaging arrangement that includes patient support pallet 417 that is translatable along a longitudinal axis 418, and in which gamma camera 414 is movable along trajectory 416 that is comprised within a plane that transversely intersects longitudinal axis 418. Thus, in contrast to Fig. 3, in the arrangement of Fig. 4 trajectory 416 is comprised within a plane that transversely intersects longitudinal axis 418. This offers alternative access for performing nuclear imaging of an object positioned on patient support pallet 417. Advantageously, this configuration can be used to provide nuclear imaging from different angular positions to longitudinal axis 318. Preferably trajectory 416 includes an arc of rotation. Such an arc can be used to reduce the change in distance from gamma detector 414 to the center of an object in the region of interest during nuclear imaging and thereby minimize depth-dependent scatter effects in the region of interest. Alternatively trajectory 416 may be a straight line. Optionally gamma camera 414 is attached to a gamma camera c-arm 421 and the arc of rotation is defined by a movement of the gamma camera along the gamma camera c-arm.

Optionally, x-ray c-arms 313, 413 in Fig. 3 and Fig. 4 respectively may include x-ray c-arm support 320, 420 which may be configured to rotate x-ray c-arm 313, 413 about x-ray c-arm axis 319, 419. X-ray c-arm support may also be configured to rotate the c-arm itself. X-ray c-arm support 320, 420 may include a swivel bearing or a sliding joint or geared track to permit such movement. X-ray c-arm axis 319, 419 and the corresponding longitudinal axis 318, 418 are mutually parallel. Moreover, in the Figures, x-ray c-arm axis 319, 419 is parallel to the y-axis. X-ray c-arm support 320, 420 may thus be used to move c-arm 313, 413 in the direction of the dotted arrows in order to achieve the rotation in the illustrated x-z plane. X-ray c-arm support 320, 420 may for example include a gear mechanism that engages with teeth attached to the c-arm in order to effect this movement. In so doing more flexible positioning of the x-ray and nuclear cameras can be achieved. The term parallel as used throughout this specification is intended to mean within approximately ±10 degrees, or within ±5 degrees of exactly parallel.

X-ray c-arms 313, 413 in Fig. 3 and Fig. 4 may additionally or alternatively be configured for rotation about the illustrated x-axis as-shown in order to further improve positioning flexibility.

In one arrangement, x-ray c-arm support 320, 420 and the gamma camera support structure are held in fixed mechanical relation to one another. Such an arrangement provides close correspondence between an x-ray image generated by the x-ray source and the x-ray detector, and an image generated by the gamma camera since any vibrations will tend to affect both imaging systems in a similar manner.

In summary a medical imaging arrangement has been described. In the medical imaging arrangement 100 an x-ray source 111 is attached to a first portion of an x-ray c-arm 113 and an x-ray detector 112 is attached to a second portion of the x-ray c-arm 113 in order to measure x-ray transmission along a path 115 between the x-ray source and the x-ray detector. A gamma camera 114 is movable along a trajectory 116 that intersects the path between the x-ray source and the x-ray detector. Since the gamma camera can be moved along a trajectory that intersects the path between the x-ray source and the x-ray detector, the gamma camera can be used to generate a nuclear image that closely corresponds to the same region of interest as that which is imaged by the x-ray source and detector.

## Claims

1. Medical imaging arrangement (100) comprising:
an x-ray source (111);
an x-ray detector (112);
an x-ray c-arm (113);
a gamma camera (114);
a gamma camera support structure;
wherein the x-ray source (111) is attached to a first portion of the x-ray c-arm (113) and the x-ray detector (112) is attached to a second portion of the x-ray c-arm (113) for measuring x-ray transmission along a path (115) between the x-ray source and the x-ray detector; and wherein the gamma camera (114) is movable along a linear trajectory (116) defined by the gamma camera support structure, and wherein said trajectory (116) intersects the path between the x-ray source and the x-ray detector such that the gamma camera (114) can be positioned in the path (115) between the x-ray source and the x-ray detector.

2. The medical imaging arrangement (100) according to claim 1 further comprising an x-ray c-arm support (320, 420);
wherein the x-ray c-arm (113) is mounted to the x-ray c-arm support (320, 420) for at least one of translation or rotation of the x-ray c-arm (113).

3. The medical imaging arrangement (100) according to claim 2 wherein the x-ray c-arm support (320, 420) and the gamma camera support structure are held in fixed mechanical relation to one another.

4. The medical imaging arrangement (100) according to any previous claim wherein the gamma camera support structure and the x-ray c-arm (113) are independently movable with respect to one another.

5. The medical imaging arrangement (300, 400) of any previous claim further comprising a patient support pallet (317, 417);
wherein the patient support pallet (317, 417) is translatable along a longitudinal axis (318, 418) that passes lengthwise through the patient support pallet (317, 417); and
wherein the path (315, 415) between the x-ray source (311, 411) and the x-ray detector (312, 412) is arranged transversely with respect to the longitudinal axis.

6. The medical imaging arrangement (300) of claim 5 wherein the trajectory (316) and the longitudinal axis (318) are mutually parallel.

7. The medical imaging arrangement (300) of claim 6 wherein the gamma camera is translatable with respect to the patient support pallet.

8. The medical imaging arrangement (300) of any one of claims 5-7 wherein the gamma camera support structure is supported by the patient support pallet (317).

9. The medical imaging arrangement (400) of claim 5 wherein the trajectory (416) is comprised within a plane that transversely intersects the longitudinal axis (418).

10. The medical imaging arrangement (100, 200, 300, 400) of any one of claims 1 - 9 wherein the trajectory intersects the path (115, 215, 315, 415) between the x-ray source (111, 211, 311, 411) and the x-ray detector (112, 212, 312, 412) at a point (Pint_{1,2,3,4}) that is closer to the x-ray source (111, 211, 311, 411) than to the x-ray detector (112, 212, 312, 412).

11. The medical imaging arrangement of any one of claims 5 - 10 wherein the x-ray c-arm is configured for rotation about an x-ray c-arm axis; and
wherein the x-ray c-arm axis and the longitudinal axis are mutually parallel.

## Patentansprüche

1. Medizinische Abbildungsanordnung (100) umfassend:
eine Röntgenquelle (111);
einen Röntgendetektor (112);
einen Röntgen-C-Arm (113);
eine Gammakamera (114);
eine Gammakamera-Stützstruktur;
wobei die Röntgenquelle (111) an einem ersten Abschnitt des Röntgen-C-Arms (113) angebracht ist und der Röntgendetektor (112) an einem zweiten Abschnitt des Röntgen-C-Arms (113) angebracht ist, um die Röntgendurchlässigkeit entlang eines Weges (115) zwischen der Röntgenquelle und dem Röntgendetektor zu messen; und wobei die Gammakamera (114) entlang eines linearen Verfahrwegs (116) bewegbar ist, der durch die Gammakamera-Stützstruktur definiert ist, und wobei der Verfahrweg (116) den Weg zwischen der Röntgenquelle und dem Röntgendetektor schneidet, so dass die Gammakamera (114) in dem Weg (115) zwischen der Röntgenquelle und dem Röntgendetektor positioniert werden kann.

2. Medizinische Abbildungsanordnung (100) nach Anspruch 1, weiter umfassend eine Röntgen-C-Arm-Stütze (320, 420);
wobei der Röntgen-C-Arm (113) an der Röntgen-C-Arm-Stütze (320, 420) für mindestens entweder eine Übersetzung oder eine Drehung des Röntgen-C-Arms (113) montiert ist.

3. Medizinische Abbildungsanordnung (100) nach Anspruch 2, wobei die Röntgen-C-Arm-Stütze (320, 420) und die Gammakamera-Stützstruktur in fester mechanischer Beziehung zueinander gehalten werden.

4. Medizinische Abbildungsanordnung (100) nach einem der vorstehenden Ansprüche,
wobei die Gammakamera-Stützstruktur und der Röntgen-C-Arm (113) unabhängig voneinander beweglich sind.

5. Medizinische Abbildungsanordnung (300, 400) nach einem der vorstehenden Ansprüche, weiter umfassend ein Patienten-Auflager (317, 417);
wobei das Patienten-Auflager (317, 417) entlang einer Längsachse (318, 418) übersetzbar ist, die längs durch das Patienten-Auflager (317, 417) verläuft; und
wobei der Weg (315, 415) zwischen der Röntgenquelle (311, 411) und dem Röntgendetektor (312, 412) quer zur Längsachse angeordnet ist.

6. Medizinische Abbildungsanordnung (300) nach Anspruch 5, wobei der Verfahrweg (316) und die Längsachse (318) zueinander parallel sind.

7. Medizinische Abbildungsanordnung (300) nach Anspruch 6, wobei die Gammakamera in Bezug auf das Patienten-Auflager übersetzbar ist.

8. Medizinische Abbildungsanordnung (300) nach einem der Ansprüche 5 bis 7, wobei die Gammakamera-Stützstruktur von dem Patienten-Auflager (317) gestützt wird.

9. Medizinische Abbildungsanordnung (400) nach Anspruch 5, wobei der Verfahrweg (416) innerhalb einer Ebene umfasst ist, welche die Längsachse (418) quer schneidet.

10. Medizinische Abbildungsanordnung (100, 200, 300, 400) nach einem der Ansprüche 1 bis 9, wobei der Verfahrweg den Weg (115, 215, 315, 415) zwischen der Röntgenquelle (111, 211, 311, 411) und dem Röntgendetektor (112, 212, 312, 412) an einem Punkt (Pint_{1, 2,3,4}) schneidet, der näher an der Röntgenquelle (111, 211, 311, 411) als an dem Röntgendetektor (112, 212, 312, 412) liegt.

11. Medizinische Abbildungsanordnung nach einem der Ansprüche 5 bis 10, wobei der Röntgen-C-Arm zur Drehung um eine Röntgen-C-Arm-Achse konfiguriert ist; und
wobei die Röntgen-C-Arm-Achse und die Längsachse zueinander parallel sind.

## Revendications

1. Agencement d'imagerie médicale (100) comprenant :
une source de rayons X (111) ;
un détecteur de rayons X (112) ;
un arceau de radiographie (113)
une caméra gamma (114) ;
une structure de support de la caméra gamma ;
dans lequel la source de rayons X (111) est fixée à une première partie de l'arceau de radiographie (113) et le détecteur de rayons X (112) est fixé à une seconde partie de l'arceau de radiographie (113) pour mesurer la transmission des rayons X le long d'un trajet (115) entre la source de rayons X et le détecteur de rayons X ; et
dans lequel la caméra gamma (114) peut être déplacée le long d'une trajectoire linéaire (116) définie par la structure de support de la caméra gamma et dans lequel ladite trajectoire (116) coupe le trajet entre la source de rayons X et le détecteur de rayons X de sorte que la caméra gamma (114) puisse être positionnée dans le trajet (115) entre la source de rayons X et le détecteur de rayons X.

2. Agencement d'imagerie médicale (100) selon la revendication 1, comprenant en outre un support d'arceau de radiographie (320, 420) ;
dans lequel l'arceau de radiographie (113) est monté sur le support d'arceau de radiographie (320, 420) pour au moins effectuer l'une d'une translation ou d'une rotation de l'arceau de radiographie (113).

3. Agencement d'imagerie médicale (100) selon la revendication 2, dans lequel le support d'arceau de radiographie (320, 420) et la structure de support de la caméra gamma sont maintenus en relation mécanique fixe mutuellement.

4. Agencement d'imagerie médicale (100) selon l'une quelconque des revendications précédentes, dans lequel la structure de support de la caméra gamma et l'arceau de radiographie (113) sont indépendamment mobiles l'un(e) par rapport à l'autre.

5. Agencement d'imagerie médicale (300, 400) selon l'une quelconque des revendications précédentes, comprenant en outre une palette de support de patient (317, 417) ;
dans lequel la palette de support de patient (317, 417) peut effectuer une translation le long d'un axe longitudinal (318, 418) qui passe longitudinalement à travers la palette de support de patient (317, 417) ; et
dans lequel le trajet (315, 415) entre la source de rayons X (311, 411) et le détecteur de rayons X (312, 412) est agencé transversalement par rapport à l'axe longitudinal.

6. Agencement d'imagerie médicale selon la revendication 5, dans lequel la trajectoire (316) et l'axe longitudinal (318) sont mutuellement parallèles.

7. Agencement d'imagerie médicale (300) selon la revendication 6, dans lequel la caméra gamma peut effectuer une translation par rapport à la palette de support de patient.

8. Agencement d'imagerie médicale (300) selon l'une quelconque des revendications 5-7, dans lequel la structure de support de la caméra gamma est supportée par la palette de support de patient (317).

9. Agencement d'imagerie médicale (400) selon la revendication 5, dans lequel la trajectoire (416) est comprise dans un plan qui coupe transversalement l'axe longitudinal (418).

10. Agencement d'imagerie médicale (100, 200, 300, 400) selon l'une quelconque des revendications 1-9, dans lequel la trajectoire coupe le trajet (115, 215, 315, 415) entre la source de rayons X (111, 211, 311, 411) et le détecteur de rayons X (112, 212, 312, 412) en un point (Pint_{1, 2,3,4}) qui est plus proche de la source de rayons X (111, 211, 311, 411) que le détecteur de rayons X (112, 212, 312, 412).

11. Agencement d'imagerie médicale selon l'une quelconque des revendications 5-10, dans lequel l'arceau de radiographie est configuré pour une rotation autour d'un axe de l'arceau de radiographie ; et
dans lequel l'axe de l'arceau de radiographie et l'axe longitudinal sont mutuellement parallèles.
